Europäisches Patentamt

European Patent Office

Office européen des brevets

(19)

(11) Veröffentlichungsnummer : **0 010 244**
**B1**

(12)                    # EUROPÄISCHE PATENTSCHRIFT

(45) Veröffentlichungstag der Patentschrift :
30.09.81

(51) Int. Cl.³ : **C 07 C143/58**

(21) Anmeldenummer : **79103839.1**

(22) Anmeldetag : **08.10.79**

(54) Verfahren zur Herstellung von 4-Nitro-4'-amino-diphenylamin-2-sulfonsäure und 2-Nitro-4'-amino-diphenylamin-4-sulfonsäure.

(30) Priorität : **13.10.78 DE 2844610**

(43) Veröffentlichungstag der Anmeldung :
**30.04.80 (Patentblatt 80/09)**

(45) Bekanntmachung des Hinweises auf die Patenterteilung : **30.09.81 Patentblatt 81/39**

(84) Benannte Vertragsstaaten :
**CH DE FR GB**

(56) Entgegenhaltungen :
**BERICHTE DER DEUTSCHEN CHEMISCHEN GESELLSCHAFT, Jahrgang 41, 1908, Weinheim, DE**
**F. ULLMANN et al. : « Über Diphenyl-Amin-Derivate », Seiten 3744-3755**

**DE - C - 86 250**

(73) Patentinhaber : **HOECHST AKTIENGESELLSCHAFT**
**Postfach 80 03 20**
**D-6230 Frankfurt/Main 80 (DE)**

(72) Erfinder : **Arndt, Otto, Dr.**
**Frankfurter Strasse 38**
**D-6238 Hofheim am Taunus (DE)**
Erfinder : **Tronich, Wolfgang, Dr.**
**Martin-Wohmann-Strasse 27**
**D-6238 Hofheim am Taunus (DE)**

# Verfahren zur Herstellung von 4-Nitro-4'-amino-diphenylamin-2-sulfonsäure und 2-Nitro-4'-amino-diphenylamin-4-sulfonsäure

4-Nitro- 4'-amino- diphenylamin-2-sulfonsäure, die daraus durch Reduktion zugängliche 4,4'-Diamino-diphenylamin-2-sulfonsäure und das daraus durch Desulfonierung herstellbare 4,4'-Diamino-diphenylamin sowie die entsprechenden Isomeren, in denen die Nitro- bzw. die zweite Aminogruppe in 2-Stellung stehen und die Sulfogruppe an die 4-Stellung geknüpft ist, sind Zwischenprodukte für Farbmittel.

Es ist bekannt, 4-Nitro-4'-amino-diphenylamin-2-sulfonsäure durch Kondensation des Natriumsalzes der 6-Chlor-3-nitro-benzolsulfonsäure mit 1,4-Phenylendiamin in Wasser in Gegenwart von Säureakzeptoren wie Natronlauge, Natriumcarbonat oder Natriumacetat und daraus durch anschließende Reduktion der Nitrogruppe die 4,4'-Diamino-diphenylamin-2-sulfonsäure herzustellen (Berichte der Deutschen Chemischen Gesellschaft 41 (1908) 3744-3755, insbesondere 3752-3753). Dort ist auch die Abspaltung der Sulfogruppe mit Schwefelsäure beschrieben.

Es hat sich jedoch gezeigt, daß unter diesen Kondensationsbedingungen als Nebenprodukt das Kondensationsprodukt aus 2 Mol 6- bzw. 4-Chlor-3-nitro-benzolsulfonsäure mit 1 Mol 1,4-Phenylendiamin gebildet wird, das bei der Reduktion in das entsprechende Diamin übergeführt wird. Dieses aus dem Biskondensationsprodukt erhaltene Diamin und die Spaltprodukte des Biskondensationsproduktes stellen störende Verunreinigungen des erwünschten Diamins dar, weshalb zur Abtrennung dieser Nebenprodukte eine zusätzliche Aufarbeitungsstufe erforderlich ist (H. E. Fierz-David u. L. Bangley, Grundlegende Operationen der Farbenchemie Springer-Verlag 1952, S. 97.).

Selbst wenn man versucht, die Bildung des Biskondensationsproduktes durch eine Überschuß an 1,4-Phenylendiamin zurückzudrängen, so werden doch noch etwa 6-7 Gew.-% des Biskondensationsproduktes gebildet. Außerdem ist eine Entfernung des im großen Überschuß eingesetzten 1,4-Phenylendiamins aus dem Abwasser erforderlich.

Bei der Kondensation wird Salzsäure frei, die durch die Säureakzeptoren in Kochsalz übergeführt wird. Außerdem können technische Qualitäten des eingesetzten Natriumsalzes Kochsalz enthalten. Das Reaktionsmedium ist also — zumindest nachdem die Kondensation in Gang gekommen ist — eine Kochsalzlösung.

Überraschenderweise wurde nun gefunden, daß die Bildung des Biskondensationsproduktes praktisch vollständig unterdrückt werden kann, wenn das Reaktionsmedium für die Kondensationsreaktion eine gesättigte wäßrige Kochsalzlösung ist. Gegenstand der Erfindung ist somit ein Verfahren zur Herstellung von 4-Nitro-4'-amino-diphenylamin-2-sulfonsäure und 2-Nitro-4'-amino-diphenylamin-4-sulfonsäure bzw. ihrer Natriumsalze durch Kondensation des Natriumsalzes der 6- bzw. 4-Chlor-3-nitro-benzolsulfonsäure in einem wäßrigen kochsalzhaltigen Medium, das dadurch gekennzeichnet ist, daß dieses Medium eine gesättigte Kochsalzlösung ist. Besonders vorteilhaft ist es, als Kondensationsmedium die Mutterlauge einer vorhergegangenen Kondensationsreaktion einzusetzen.

Überraschenderweise zeigen andere Alkali- und Erdalkalichloride diesen vorteilhaften Einfluß nicht ; ebenso weist Natriumsulfat eine gegenüber Natriumchlorid deutlich abgeschwächte Wirkung auf.

Beim erfindungsgemäßen Verfahren wird also durch die Unterdrückung der Bildung des Biskondensationsprodukts nicht nur der Ausbeuteverlust vermieden, sondern es wird auch ein Produkt erhalten, das nach der Reduktion und gegebenenfalls Desulfonierung unmittelbar als Farbenvorprodukt eingesetzt werden kann. So lassen sich beispielsweise in einem nach Desulfonierung (gemäß US-PS 2 022 889) erhaltenen 4,4'-Diamino-diphenylamin-sulfat im Dünnschichtchromatogramm weder das Biskondensationsprodukt noch dessen Spaltprodukte nachweisen.

Ein weiterer Vorteil des erfindungsgemäßen Verfahrens liegt darin, daß die 4-Nitro-4'-amino-diphenylamin-2-sulfonsäure bzw. 2-Nitro-4'-amino-diphenylamin-4-sulfonsäure als Natriumsalz anfällt, das in der gesättigten Natriumchloridlösung schwerlöslich ist. Dadurch kann die nach dem Stand der Technik erforderliche Fällung des Produktes mit Salzsäure entfallen, die den weiteren Nachteil hatte, daß für eine anschließende katalytische Reduktion mit Nickelkatalysatoren eine Neutralisation erforderlich wurde, da diese Katakysatoren säureempfindlich sind.

Bei dem erfindungsgemäßen Verfahren können die Komponenten im stöchiometrischen Molverhältnis oder ein geringer Überschuß an 1,4-Phenylendiamin eingesetzt werden.

Im folgenden werden bevorzugte Ausgestaltungen des erfindungsgemäßen Verfahrens näher beschrieben :

Das technisch feuchte Natriumsalz der 6- bzw. 4-Chlor-3-nitro-benzolsulfonsäure, das gegebenenfalls aus dem Herstellungsprozeß bereits einen gewissen Natriumchloridgehalt besitzen kann, wird in die Lösung von 1,4-Phenylendiamin, das zweckmäßigerweise in einem etwa 10 %igen Überschuß eingesetzt wird, in gesättigter Natriumchloridlösung bei erhöhter Temperatur, zweckmäßig 80-100 °C, insbesondere etwa 95 °C eingetragen. Hierbei genügt es, die gesättigte Natriumchlorid-Lösung für den ersten Ansatz aus Natriumchlorid und Wasser herzustellen, da für die nachfolgenden Ansätze zweckmäßig die Mutterlauge des vorausgegangenen Ansatzes eingesetzt wird. Es entsteht somit keine zusätzliche Belastung des Abwassers.

Die bei der Kondensation entstehende Salzsäure wird durch Säureakzeptoren, die zweckmäßigerweise mit der Salzsäure Natriumchlorid bilden, also Natriumhydroxid, Natriumcarbonat oder Natriumhydrogencarbonat, abgefangen, wobei der pH-Wert zweckmäßig zwischen 8 und 10, vorzugsweise bei 9 gehalten wird.

Aus dem entstehenden Natriumsalz der 4-Nitro-4'-amino-diphenylamin-2-sulfonsäure bzw. 2-Nitro-4'-amino-diphenylamin-4-sulfonsäure kann mit Salzsäure das schwerlösliche innere Salz hergestellt und dieses durch Filtration isoliert werden. Eine besonders bevorzugte Ausführungsform des erfindungsgemäßen Verfahrens besteht jedoch darin, daß man das in der gesättigten Natriumchlorid-Lösung schwerlösliche Natriumsalz direkt durch Filtration bei tieferer Temperatur, zweckmäßig bei Zimmertemperatur isoliert.

Sofern mit einer Mineralsäure, zweckmäßig Salzsäure, die freie Sulfosäure ausgefällt wird, wird sie nach der Filtration mit Wasser mineralsäure- und chloridfrei gewaschen. Wenn das Natriumsalz isoliert wird, wird dieses mit gesättigter Natriumchloridlösung gewaschen.

Das Kondensationsprodukt fällt in einer solchen Reinheit an, daß es nach üblicher Reduktion, beispielsweise mit Eisen oder katalytisch mit Wasserstoff, gegebenenfalls nach anschließender Desulfonierung, unmittelbar als Farbenvorprodukt verwendet werden kann.

In den folgenden Beispielen beziehen sich Teile und Prozentangaben auf das Gewicht, sofern nichts anderes angegeben ist.

Beispiel 1

519 Teile des Natriumsalzes der 6-Chlor-3-nitro-benzolsulfonsäure (2 Mol) werden in eine 95 °C heiße Lösung von 240 Teilen 1,4-Phenylendiamin (2,22 Mol) in 5 700 Teilen gesättigter wäßriger Kochsalzlösung (26 % NaCl) eingetragen. Durch Zugabe von 250 Teilen 33 %iger Natronlauge wird die bei der — sofort einsetzenden — Kondensation freiwerdende Salzsäure neutralisiert und der pH-Wert ständig bei 9 gehalten. Die Umsetzung dauert ca. 6 Stunden.

Nach Abschluß der Kondensationsreaktion wird das Reaktionsgemisch bei 70 °C mit 240 Teilen 30 %iger Salzsäure auf pH 1,5 gestellt. Die ausgefallene freie Sulfonsäure wird filtriert und mit Wasser chloridfrei gewaschen. Man erhält ca. 1 000 Teile Feuchtprodukt, das 620 Teile Sulfonsäure enthält. Das wasserfeuchte Produkt wird in 1 700 Teile Wasser eingetragen und die Mischung mit 200 Teilen 33 %iger Natronlauge auf pH 10 gestellt, dann, beispielsweise mit Phosphorsäure, auf pH 7,0 gepuffert. Dieses Gemisch wird in einen 5 Liter Edelstahl-Hydrierautoklav überführt, der 10 Teile Nickelkatalysator (55 % Ni auf Kieselgur) enthält und dieser mit Stockstoff gespült. Dann wird bei 70 bis 95 °C unter einem Druck von 40 bar Wasserstoff im Laufe von etwa einer halben bis einer Stunde reduziert. Der Autoklav wird entspannt, die Reaktionsmischung, die pH 8 zeigt, bei 95 °C vom Katalysator über eine Drucknutsche unter Stickstoff filtriert und der Katalysator mit heißem Wasser gewaschen.

Aus dem geklärten Filtrat (3 500 Teile) wird mit 250 Teilen 30 %iger Salzsäure die 4,4'-Diamino-diphenylamin-2-sulfonsäure bei einem pH-Wert bis 5,5 gefällt. Das Produkt wird bei 25 °C filtriert und mit Wasser gewaschen. Man erhält etwa 700 Teile Feuchtprodukt und nach dem Trocknen 519 Teile 4,4'-Diamino-diphenylamin-2-sulfonsäure mit einem Reingehalt von 98 %, entsprechend einer Ausbeute von 92 % der Theorie, bezogen auf 6-Chlor-3-nitro-benzolsulfonsäure.

Die so erhaltene feuchte Sulfonsäure wird in 1 000 Teile ca. 70 %ige Schwefelsäure eingetragen. Durch den Wassergehalt des Reaktionsproduktes sinkt die Konzentration der Schwefelsäure auf ca. 55 %. Das Gemisch wird 5-6 Stunden bei 115 °C gerührt, wobei sich eine schwarze, klare Lösung bildet. Diese Lösung läßt man dann in 2 000 Teile 50-60 °C heißes Wasser unter Rühren einlaufen, wobei das Sulfat des 4,4'-Diaminodiphenylamins ausfällt. Man filtriert das Sulfat bei 25 °C ab und wäscht es mit Wasser. Man erhält ca. 1 200 Teile Feuchtprodukt mit einem Gehalt an 319 Teilen 4,4'-Diamino-diphenylamin, entsprechend einer Ausbeute von 87 % der Theorie, bezogen auf 4,4'-Diamino-diphenylamin-2-sulfonsäure bzw. 80 % der Theorie, bezogen auf 6-Chlor-3-nitro-benzolsulfonsäure. Das Dünnschichtchromatogramm zeigt keine diazotierbaren und fluoreszenzlöschenden Nebenkomponenten.

Beispiel 2 (Vergleichsbeispiel)

Die Kondensation erfolgt wie in Beispiel 1, jedoch ohne Zusatz von Natriumchlorid, d.h. das 1,4-Phenylendiamin wird in 4 200 Teile Wasser eingetragen. Die bei der Kondensation frei werdende Salzsäure verbraucht 235 Teile 33 %ige Natronlauge. Die Fällung des Produkts erfolgt mit 315 Teilen 30 %iger Salzsäure (bis pH 1,5). Die katalytische Reduktion erfolgt wie im Beispiel 1. Man erhält nach dem Trocknen 490 Teile 4,4'-Diamino-diphenylamin-2-sulfonsäure mit einem Gehalt von 7-8 % Biskondensationsprodukt.

Die Abspaltung der Sulfonsäuregruppe erfolgt wie in Beispiel 1. Das Dünnschichtchromatogramm des 4,4'-Diamino-diphenylamin-sulfats zeigt eine starke Verunreinigung durch das Biskondensationsprodukt.

Beispiel 3

Die Kondensation erfolgt wie in Beispiel 1. Man läßt jedoch nach der abgeschlossenen Kondensation keine Salzsäure zulaufen, sondern isoliert bei 25 °C das schwerlösliche Natriumsalz der 4-Nitro-4'-amino-diphenylamin-2-sulfonsäure.

Das Produkt wird mit 1 200 Teilen gesättigter Natriumchlorid-Lösung gewaschen. Man erhält ca. 900 Teile Feuchtprodukt mit 680 Teilen Trockensubstanz mit einem Reingehalt von 596 Teilen. Das wasserfeuchte Produkt wird in 1 800 Teile Wasser eingetragen. Die dünne Suspension hat einen pH von 8,5-9, der, beispielsweise mit Phosphorsäure, auf pH 7,0 gepuffert wird.

Anschließend wird wie in Beispiel 1 zur 4,4'-Diamino-diphenylamin-2-sulfonsäure katalytisch hydriert. Man erhält nach der Isolierung ca. 600 Teile Feuchtprodukt und nach dem Trocknen 500 Teile 4,4'-Diamino-diphenylamin-2-sulfonsäure mit einem Reingehalt von 98 %, entsprechend einer Ausbeute von 88 % der Theorie, bezogen auf 6-Chlor-3-nitrobenzolsulfonsäure. Das daraus durch Abspaltung der Sulfogruppe hergestellte 4,4'-Diamino-diphenylaminsulfat hat die gleiche Qualität wie das Produkt nach Beispiel 1.

## Beispiel 4

Die Kondensation erfolgt wie in Beispiel 1, es werden jedoch anstelle der 5 700 Teile gesättigter wäßriger Natriumchloridlösung 5 800 Teile (ca. 75 %) der Mutterlauge aus einem vorausgehenden Ansatz, beispielsweise der Mutterlauge nach Beispiel 3, eingesetzt. Qualität und Ausbeute an 4,4'-Diamino-diphenylamin-2-sulfonsäure stimmen mit den Angaben von Beispiel 1 überein.

## Beispiel 5

Kondensation und Reduktion werden durchgeführt wie in Beispiel 1 beschrieben, mit dem Unterschied, daß an Stelle des Natriumsalzes von 6-Chlor-3-nitrobenzolsulfonsäure das Natriumsalz der 4-Chlor-3-nitrobenzolsulfonsäure eingesetzt wirk. Man erhält aus der Kondensation 620 Teile 2-Nitro-4'-amino-diphenylamin-4-sulfonsäure.

Das aus deren Reduktion stammende katalysatorfreie Filtrat (5 200 Teile) wird mit 575 Teilen 30 %iger Salzsäure bis pH 8,0, dann mit 2 000 Teilen Natriumchlorid versetzt. Die 2,4'-Diamino-diphenylamin-4-sulfonsäure fällt als Hydrochlorid aus und wird bei 25 °C abfiltriert.

Das Produkt enthält kein Biskondensationsprodukt aus 2 Mol 4-Chlor-3-amino-benzolsulfonsäure und 1 Mol 1,4-Phenylendiamin.

## Beispiel 6 (Vergleischsbeispiel)

Die Kondensation wird durchgeführt wie in Beispiel 2 beschrieben, also ohne NaCl, jedoch mit dem Natriumsalz der 4-Chlor-3-nitrobenzolsulfonsäure an Stelle des Natriumsalzes der 6-Chlor-3-nitrobenzolsulfonsäure.

Die Reduktion wird entsprechend dem Beispiel 1, die Isolierung entsprechend Beispiel 5 durchgeführt. Die so hergestellte 2,4'-Diamino-diphenylamin-4-sulfonsäure enthält 22 Teile des im Beispiel definierten Biskondensationsproduktes (= 5 % d.Th., bezogen auf das Hauptprodukt).

## Ansprüche

1. Verfahren zur Herstellung von 4-Nitro-4'-amino-diphenylamin-2-sulfonsäure und 2-Nitro-4'-amino-diphenylamin-4-sulfonsäure bzw. ihrer Natriumsalze durch Kondensation des Natriumsalzes der 6- bzw. 4-Chlor-3-nitro-benzolsulfonsäure mit 1,4-Phenylendiamin in einem wäßrigen kochsalzhaltigen Medium, dadurch gekennzeichnet, daß das Reaktionsmedium eine gesättigte Kochsalzlösung ist.

2. Verfahren nach Anspruch 1, dadurch gekennzeichnet, daß als Reaktionsmedium die Mutterlauge einer vorhergegangenen Kondensationsreaktion eingesetzt wird.

3. Verfahren nach Anspruch 1 und 2, dadurch gekennzeichnet, daß das Produkt als Natriumsalz durch Kühlen der Reaktionsmischung isoliert wird.

4. Verfahren nach Anspruch 1 und 2, dadurch gekennzeichnet, daß das Produkt als freie Säure durch Ansäuern der Reaktionsmischung isoliert wird.

5. Verfahren nach Anspruch 1 bis 4, dadurch gekennzeichnet, daß der pH-Wert der Reaktionsmischung zwischen 8 und 10 gehalten wird.

6. Verfahren nach Anspruch 1 bis 5, dadurch gekennzeichnet, daß durch Zugabe von Natriumhydroxid, -carbonat oder -hydrogencarbonat der pH-Wert der Reaktionsmischung zwischen 8 und 10 gehalten wird.

7. Verfahren nach Anspruch 1 bis 6, dadurch gekennzeichnet, daß die Kondensation bei 80 bis 100 °C durchgeführt wird.

8. Verfahren nach Anspruch 1 bis 7, dadurch gekennzeichnet, daß das Phenylendiamin in einem 10 %igen Überschuß eingesetzt wird.

## Claims

1. A process for the preparation of 4-nitro-4'-amino-diphenylamine-2-sulfonic acid and 2-nitro-4'-amino-diphenylamine-4-sulfonic acid and their sodium salts by condensation of the sodium salt of 6- respectively 4-chloro-3-nitro-benzene sulfonic acid with 1,4-phenylene diamine in an aqueous medium containing sodium chloride, characterized in that the reaction medium is a saturated aqueous solution of sodium chloride.

2. A process according to claim 1, characterized in that the reaction medium is the mother liquor of a preceding condensation reaction.

3. A process according to claims 1 and 2, characterized in that the product is isolated as the sodium salt by cooling the reaction solution.

4. A process according to claims 1 and 2, characterized in that the product is isolated in form of the free acid by acidifying the reaction medium.

5. A process according to claims 1 to 4, characterized in that the pH-value of the reaction mixture is maintained between 8 and 10.

6. A process according to claims 1 to 5, characterized in that the pH-value of the reaction mixture is maintained between 8 and 10 by addition of sodium hydroxide, sodium carbonate or sodium hydrogencarbonate.

7. A process according to claims 1 to 6, characterized in that the condensation is effected at 80 to 100 °C.

8. A process according to claims 1 to 7, characterized in that the 1,4-phenylene diamine is reacted in an excess of 10 %.

## Revendications

1. Procédé de préparation de l'acide nitro-4 amino-4′ diphénylamine-sulfonique-2 et de l'acide nitro-2 amino-4′ diphénylamine-sulfonique-4, ou de leurs sels sodiques, par condensation du sel sodique de l'acide chloro-6 ou chloro-4 nitro-3 benzène-sulfonique avec la phénylène-diamine-1,4 dans un milieu aqueux contenant du chlorure de sodium, procédé caractérisé en ce que le milieu réactionnel est une solution saturée de chlorure de sodium.

2. Procédé selon la revendication 1, caractérisé en ce qu'on utilise, comme milieu réactionnel, la liqueur-mère provenant d'une réaction de condensation antérieure.

3. Procédé selon l'une des revendications 1 et 2, caractérisé en ce qu'on isole le produit à l'état de sel sodique par refroidissement du mélange réactionnel.

4. Procédé selon l'une des revendications 1 et 2, caractérisé en ce qu'on isole le produit à l'état d'acide libre par acidification du mélange réactionnel.

5. Procédé selon l'une quelconque des revendications 1 à 4, caractérisé en ce qu'on maintient le pH du mélange réactionnel entre 8 et 10.

6. Procédé selon l'une quelconque des revendications 1 à 5, caractérisé en ce qu'on maintient le pH du mélange réactionnel entre 8 et 10 par addition d'hydroxyde de sodium, de carbonate de sodium ou d'hydrogénocarbonate de sodium.

7. Procédé selon l'une quelconque des revendications 1 à 6, caractérisé en ce qu'on effectue la condensation à une température comprise entre 80 et 100 °C.

8. Procédé selon l'une quelconque des revendications 1 à 7, caractérisé en ce qu'on utilise la phénylène-diamine en un excès de 10 %.